# EUROPEAN PATENT APPLICATION

(11) **EP 1 693 348 A1**
(43) Date of publication of application: **23.08.2006**
(21) Application number: 06003433.7
(22) Date of filing: 20.02.2006
(51) Int. Cl.: C03B 37/018

(54) **Multimode optical fiber and method for manufacturing same**

(30) Priority: 22.02.2005 US 63107
(71) Applicant: Furukawa Electric North America Inc. (a Delaware Corporation), Norcross GA 30071 (US)
(72) Inventor: Baumgart, Jerry W., Lawrenceville Georgia 30043 (US); Oulundsen, George E., III, Belchertown Massachusetts 01007 (US); Yan, Man Fei, Berkeley Heights New Jersey 07922 (US)
(74) Representative: Schoppe, Fritz

(57) **Abstract**

An improved MCVD process reduces a rippling structure in the refractive-index profile of a graded-index, multiple-mode optical fiber by incorporating N₂O, CO, or NF₃ gas in the gas stream during deposition of a soot sub-layer from which the optical fiber is formed. The soot sub-layer is sintered to form a glass sub-layer during deposition of a subsequent soot sub-layer. A dopant species is incorporated in each soot sub-layer during deposition. Fibers made from the doped glass sub-layers have a graded refractive-index profile that is near-parabolic in shape and that has significantly reduced rippling compared to profiles observed for fibers prepared conventionally.

## Description

### BACKGROUND OF THE INVENTION

**Field of the Invention**

The present invention relates generally to an improved multimode (MM) optical fiber made by a chemical vapor deposition (CVD) process. More particularly, the present invention relates to a graded-index (GI) MM optical fiber made by a modified CVD (MCVD) process.

**Related Art**

Optical communications systems generally operate in the visible or near-visible region of the electromagnetic spectrum, and generally utilize cladded glass fibers as the transmission medium. Such fibers usually have at least two sections: a core and a cladding layer surrounding the core. Generally, the cladding layer has a lower refractive index (referred to herein as "index") relative to the core. The difference in index between the core and the cladding layer may be in the range of about 0.005 to 0.05.

Fibers for single-mode transmission are characterized by a core that is sufficiently small in diameter to efficiently accommodate only a first-order mode. Fibers for MM transmission are characterized by a core that is sufficiently large in diameter to accommodate multiple modes (typical MM core diameters are in the range of 50 *µ*m to 100 *µ*m and typical cladding diameters are in the range of 125 *µ*m to 150 *µ*m). Presently, fibers for MM transmission are of interest because their larger core diameters facilitate splicing and enable an efficient energy coupling to devices such as source and repeater devices.

The presence of multiple modes in a transmission line is associated with dispersion effects, which usually cause a smearing or spreading of the transmitted signals due to the different velocities of the different multiple modes. That is, a signal pulse transmitted from location A will be received at location B over a time interval or spread corresponding to the difference between the arrival times of the fastest mode and the slowest mode. Pulse spreading adversely affects optical communications by reducing the bandwidth of signal transmissions.

The effects of mode dispersion have been reduced through use of a continuously focusing GI fiber. Such a fiber has an index that is graded from a high value at the center of its core to a value that decreases with radial distance from the center of the core to the core-cladding interface. Fundamental-mode signals generally are confined to the highest index (lowest velocity) region. Higher-order-mode signals generally travel in lower index (higher velocity) regions.

Many conventional processes for producing cladded glass fibers involve the use of vapor source materials. Typically, the fibers are formed from gaseous silicon-bearing compounds, such as silicon chlorides and silicon hydrides. The compounds are reacted with oxygen to produce or deposit one or more glass layers of a preform from which the fibers are drawn. To tailor the index of the fibers, desired dopants are incorporated in the glass layer(s) by, for example, reacting a gaseous dopant-bearing compound with oxygen during formation of the glass layer(s), or coating the glass layer(s) with a liquid dopant-bearing solution and then heat treating the coated layer(s), as discussed in more detail below. Dopant materials include compounds of, for example, fluorine and/or boron for lowering the index; and germanium, titanium, aluminum, and/or phosphorus for increasing the index. In order to produce GI fibers, grading of the index may be achieved by selectively varying the type of dopant and/or the amount of dopant incorporated during vapor deposition of a plurality of glass layers. Solution doping, however, is not a high-throughput, cost-efficient way to produce GI fibers.

In a conventional CVD process for forming optical fibers, a gaseous silicon-bearing compound and oxygen are passed over a heated surface on which the gases react to form a glassy silicon-oxide material. The surface usually is the interior surface of a glass tube. Optionally, a gaseous dopant-bearing compound also may be included in the reaction to form a doped-silicon-oxide glass material. The temperature of the heated surface and the flow rates of the gases used in the reaction are adjusted and controlled so that the reaction proceeds solely via the heated surface (i.e., heterogeneously), such that the deposited material is a continuous layer of glass.

In another conventional process for forming optical fibers, commonly referred to as a "soot" process, a gas stream of precursor reactants, which includes oxygen, a gaseous silicon-bearing compound, and optionally a gaseous dopant-bearing compound, is passed through a glass tube. A moving heat source, such as a flame, passes along the tube to create a moving hot zone that heats the tube and the portion of the gas stream flowing through the moving hot zone. The precursor reactants passing through the hot zone undergo a homogeneous reaction in which glass particles (also referred to herein as "soot") are formed in the gas phase, i.e., without an intermediary surface. The gas stream moves the glass particles and causes them to deposit on the tube downstream from where they are formed. The soot that adheres to the tube subsequently is sintered to form a layer of glass on the tube.

The conventional CVD process produces glass layers that are of higher purity than those produced by the conventional soot process, but generally requires longer deposition times. The soot process, on the other hand, is prone to contamination. Also, glass layers formed by the soot process tend to suffer from hydration, which results in water-absorption-related peaks in the optical characteristics of fibers made using the soot process, and which in turn affects signal transmission in the infrared region of the electromagnetic spectrum of such fibers.

Optical fibers typically are formed (drawn) from preforms made by collapsing the glass tubes in which the doped or undoped glass layers are deposited. The fibers usually have a radial composition that is the same as that of the preforms from which they are drawn, including compositionally distinct regions corresponding to the core and the cladding layer.

Conventional MCVD processes combine aspects of the CVD process and the soot process. In a conventional MCVD process for producing a preform, one or more reagent gases, such as SiCl₄ and GeCl₄, are introduced along with oxygen into the interior of a rotating glass tube. The tube is externally heated to cause a heterogeneous oxidation reaction of the reagent gas(es) and the oxygen on the heated internal wall of the glass tube (CVD process), as well as a homogeneous oxidation reaction of the reagent gas(es) with the oxygen inside the tube (soot process). The soot formed by the reaction deposits as a thin, porous layer on the interior surface of the tube. Multiple depositions or MCVD passes may be used to form the cladding layer and/or the core of the preform. Material for the cladding layer is deposited before material for the core. Dopants may be incorporated in the core and/or the cladding layer by introducing them in gaseous form (e.g., GeCl₄) to the reaction.

Not all desirable dopants are available in gaseous form at ambient conditions. For example, certain rare-earth elements do not exist as gaseous compounds at room temperature. Such dopants may be incorporated through a solution-doping process. Solution doping involves soaking the porous soot layer with a solution that contains the desired dopant, and then draining the solution to leave behind a residue that contains the desired dopant. The residue-covered soot layer is sintered to incorporate the desired dopant in the resulting glass layer.

Solution doping of glass layers formed by a conventional MCVD process entails a number of difficulties. Sintering of the glass layers prior to soaking is not desirable because, among other things, it reduces the degree of incorporation of the desired dopant. Also, variations in porosity or degree of sintering along the glass tube produce unwanted variations in the concentration and uniformity of the solution residue, which, in turn, result in variations in dopant concentration from preform to preform as well as variations in dopant concentration along the length of fibers drawn from such preforms.

State-of-the-art MM fibers are designed to have a graded index, with a refractive-index profile that increases in a near-parabolic manner from the edge of the core-cladding boundary to the center of the core. This usually is accomplished by forming multiple glass layers (sub-layers) of progressively varying dopant concentration. As will be appreciated by one of ordinary skill in the art, achieving a reproducible near-parabolic profile through solution doping is wrought with difficulties. Further, because the individual sub-layers need to be separately soaked, solution doping is not an efficient process for achieving GI fibers with near-parabolic refi-active-index profiles.

For doping through oxidation of dopant-bearing gases, in the conventional MCVD process, the resulting preforms often have a dopant-concentration profile that has an undesirable layered structure, with ripples demarcating the layers formed in the multiple deposition passes used to produce the core sections of the preforms. Such non-uniformity in dopant concentration is reflected in a refractive-index profile that has a similar undesirable layered structure or rippling. Although the amplitude of the ripples usually are below the threshold at which optical transmission parameters are affected to the point where the preforms are unusable for GI MM optical fibers, the ripples nevertheless may limit the bandwidth of the fibers and also the ability to reliably predict accurate index information for the preforms. Currently, it is necessary to obtain index data from a fiber before any reliable tuning of its refractive-index profile is possible.

The ripples in dopant concentration, and hence index, are believed to be caused by a combination of a number of effects. First, in the case of Ge-doped silica glass, silica particles having different GeO₂ concentrations result from thermal differences across the gas phase during the oxidation reaction. The equilibrium of the GeO₂ oxidation reaction is such that, at deposition temperatures used in the conventional MCVD process, soot that forms closer to the substrate, i.e., closer to the interior surface of the glass tube, which is at a relatively higher temperature, has lower concentration of GeO₂ than soot formed farther away from the substrate, i.e., closer to the center of the tube, which is at a relatively lower temperature. Therefore, in a preform produced by the conventional MCVD process, the GeO₂ concentration within each soot sub-layer decreases radially toward the center of the tube (and thus towards the center of the resulting core section).

The center of the tube generally has a lower temperature than that near the interior surface of the tube. This radial temperature distribution affects the index of particles formed at different radial positions inside the tube. Also, within a given soot sub-layer deposited by a single MCVD pass, i.e., a single pass of the hot zone along the length of the tube, the bottom (far downstream) region is formed of particles oxidized near the center of the tube; and the top (near downstream) region is formed of particles oxidized near the interior surface of the tube. This occurs because particles that are oxidized near the center of the tube center travel at a faster axial velocity and thus travel farther downstream to deposit closer to the bottom, whereas particles that are oxidized near the interior surface of the tube travel at a slower axially velocity and deposit closer to the top.

Another effect occurs when a previously deposited soot sub-layer is sintered during a subsequent MCVD pass. That is, during each pass of the hot zone along the length of the glass tube to deposit a soot sub-layer, a small amount of GeO₂ from a surface region of a previously deposited sub-layer is "burned off." This gives rise to a sub-layer (the previously deposited sub-layer) in which the concentration of GeO₂ decreases radially toward the center of the tube. Thus, a peak or ripple in the refractive-index profile is present for each sub-layer whose surface concentration of GeO₂ is burned off during an MCVD pass.

In view of the above, there is a need in the art for a cost-efficient MCVD process that reproducibly and controllably forms glass layers for GI MM fibers with a desired near-parabolic refractive-index profile having a reduced ripple structure suitable for high-bandwidth applications.

### SUMMARY OF INVENTION

To achieve consistent high-bandwidth optical fibers needed for today's bandwidth demand, it is necessary to precisely control the refractive-index profile of the fibers and therefore to control the process for depositing the glass layers from which the fibers are formed. The present invention relates to an improved MCVD process for depositing glass layers suitable for use in MM fibers in which the presence of ripples in the refractive-index profile thereof, resulting from non-uniformities in the dopant concentration, is significantly reduced compared with MM fibers made using the conventional MCVD process.

One method that has been used to reduce the degree of rippling has been to decrease the thickness of each deposited sub-layer. This method, however, requires an increase in the number of deposition passes to achieve a desired preform size. Clearly, such a conventional technique decreases productivity, because as the number of passes increases the total deposition time increases.

The improved MCVD process of the present invention reduces the rippling effect, i.e., the variations in the refractive-index profile of the resulting glass fibers, without increasing the number of deposition passes. Therefore, the present invention provides a cost-efficient method for producing preforms used to make GI MM fibers for high-bandwidth applications. The improved MCVD process of the present invention produces soot having a small particle size and a uniform dopant concentration, which results in a significantly smoother near-parabolic refractive-index profile than what is possible conventionally.

According to an embodiment of the present invention, controlled grading of the index of a glass fiber is accomplished by an MCVD process in which the deposited soot layers are doped during soot formation by a gaseous dopant-bearing compound, which results in glass layers (formed from the soot layers) having a higher index than that of pure SiO₂ quartz. Preferably, the dopant of choice is germanium. Doping is achieved by delivering to the interior of a starting quartz or glass tube (substrate tube) a vapor stream of O₂ vapor saturated with GeCl₄ and SiCl₄ gases in proportions suitable to yield a desired index for the soot layer being deposited. The vapor stream also includes N₂O gas. Optionally, instead of N₂O, NF₃ or CO may be used. Heat is supplied to the outside of the substrate tube and creates a heat zone within the tube. Heat within the heat zone initiates a reaction inside the tube that converts the SiCl₄ and the GeCl₄ to, respectively, SiO₂ and GeO₂ soot, which deposits downstream from the heat. As the heat zone traverses the tube toward the soot layer just deposited, the heat is sufficient to cause the soot layer to sinter into a high purity glass layer containing the proper concentration of GeO₂ to give rise to the desired index for that particular glass layer. Many successive glass layers are deposited, each with a unique desired index to create a multi-layer composite with the desired grading of the refractive-index profile for the particular type of MM fiber to be formed. The refractive-index profile is tunable, such that a high-bandwidth fiber tailored for a given spectral wavelength range is produced.

The presence of N₂O or NF₃ or CO during deposition of the soot layers results in an improved uniformity of the GeO₂ concentration within each resulting glass layer. It is believed that the presence of N₂O causes the soot-formation reactions to be exothermic, which improves the radial temperature uniformity within the tube. Preforms produced by the improved MCVD process of the present invention have cores with a refractive-index profile that is significantly smoother and that has smaller index-ripples than preforms produced using the conventional MCVD process, for similarly sized preforms made from the same number of MCVD passes.

### BRIEF DESCRIPTION OF THE DRAWINGS

The present invention will be more readily understood from the detailed description of the preferred embodiment(s) presented below considered in conjunction with the attached drawings, of which:

FIG. 1 is a flow chart of a process for producing GI MM optical fibers with reduced rippling, according to an embodiment of the present invention;

FIG. 2 is a schematic diagram of an apparatus for performing the process of FIG. 1;

FIG. 3 is a chart showing the variation in index along a radius of a 200-pass preform made by a conventional MCVD process;

FIG. 4 is a chart showing the variation in index along a radius of a 250-pass preform made by a conventional MCVD process;

FIG. 5 is a chart showing the variation in index along a radius of a 150-pass preform made by an improved MCVD process, according to an embodiment of the present invention;

FIG. 6 is a chart showing the variation in index along a radius of a 150-pass preform made by an improved MCVD process, according to an embodiment of the present invention, in which a supply of N₂O is shut off at pass 124;

FIG. 7A is a chart showing the variation in index along a radius of a 150-pass preform made by a conventional MCVD process using a predefined deposition recipe; and

FIG. 7B is a chart showing the variation in index along a radius of a 150-pass preform made by an improved MCVD process, according to an embodiment of the present invention, using oxygen compensation and the predefined deposition recipe of the preform of FIG. 7A.

### DETAILED DESCRIPTION OF THE PREFERRED EMBODIMENTS

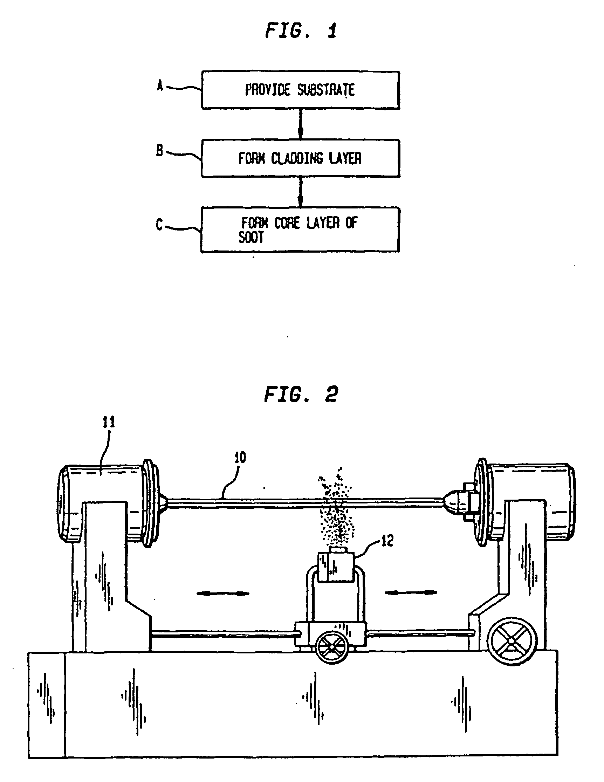

FIG. 1 is a schematic flow diagram of an improved MCVD process for making preforms for optical fibers. At step A, a substrate is provided. The substrate may be any material that is able to withstand the elevated temperatures and chemical species used in the process. For example, the substrate may be formed of a glass, a ceramic, a semiconductor material, or a high-temperature alloy. Preferably, for making preforms for optical fibers, the substrate is a hollow tube of glass. For example, as shown in FIG. 2, a tube **10** made of fused silica is used as the substrate. Typically, the tube **10** has an ID ≥6 mm and a wall thickness ≤6 mm, and may be held by a standard glass-working lathe **11.**

In step B, a cladding layer of silica soot is deposited on the substrate. The cladding layer may be formed using any known process for forming silica soot, including the soot process and the conventional MCVD process discussed above. For example, the silica soot for the cladding layer may be deposited according to the conventional MCVD process by flowing a gaseous precursor mixture into the interior of the tube **10** and applying heat to the tube's wall. The heat is sufficient to cause the gases in the precursor mixture within the interior of the tube **10** to react to form the silica soot. Preferably, the tube **10** is rotated during soot formation. One or more sub-layers of the cladding layer may be formed from a precursor mixture of, for example, SiCl₄ and O₂ gases. Optionally, the cladding layer (or any of its sub-layers) may be doped with one or more of the following species: phosphorous (by a POCl₃ precursor); germanium (by a GeCl₄ precursor), fluorine (by a C₂F₆, SF₆, or SiF₄ precursor); boron (by a BCl₃ precursor ); and aluminum (by a AlCl₃ precursor). Heating may be performed using any known movable heating source, including an oxygen-hydrogen torch **12** (shown in FIG. 2), which traverses the length of the tube **10** at a velocity of 40 to 300 mm/min and heats the tube's wall to a temperature ranging from about 1700 °C to about 2350 °C. Alternatively, heating may be performed using a reciprocating plasma torch (not shown). Preferably, the O₂ in the precursor mixture is present in an amount greater than what is necessary for a stoichiometric oxidation reaction, and helium is added to the tube **10** as a carrier gas and to assist in sintering of the sub-layer(s). The flow of helium ranges from about 50 cc/min to about 4000 cc/min. Each deposited sub-layer of soot is sintered into a glass layer when the heat source heats the tube's wall during the deposition of a subsequent sub-layer.

Optionally, as an alternative to depositing material for the cladding layer inside a starting glass tube, multiple graded-index core sub-layers, which together form a core layer, are deposited directly on the interior surface of the glass tube. (Formation of the core layer is discussed in detail below.) The tube then is collapsed to form a rod-shaped core. The rod, i.e., the core, is slid into a larger tube of pure glass, which serves as material for a cladding layer on the core. The larger tube then is collapsed onto the core to form a cladding layer around a graded-index core.

In step C, a core layer of silica soot is formed on the cladding layer or, if step B is omitted, on the interior surface of the starting glass tube. The core layer is deposited in a process similar to that used for depositing the cladding layer (described above), except that the gaseous precursor mixture includes SiCl₄, O₂, and at least one gaseous dopant-bearing compound such as, for example, GeCl₄ and POCl₃, which oxidize to form GeO₂ and P₂O₅ soot, respectively. Additionally, N₂O gas is added to the precursor mixture to improve the uniformity of the dopant concentration in the resulting soot. Optionally, instead of N₂O, NF₃ or CO may be used.

The N₂O (or NF₃ or CO) reacts readily with gases such as SiCl₄ and GeCl₄ at the torch temperature(s) used to deposit the core layer, which generally ranges from about 1600 °C to about 2350 °C. Preferably, the deposition temperature of the core layer ranges from about 1700 °C to about 2350 °C. Optionally, helium is added as a carrier gas to dilute the N₂O (or NF₃ or CO) and thereby increase the controllability of the oxidation reaction.

The present invention may be better understood by reference to the following example.

EXAMPLE

Multiple pure silica cladding sub-layers (in composite forming a cladding layer) are deposited in a silica tube by flowing 1.5 g/min of SiCl₄ in 850 cc/min of O₂ and 400 cc/min of He. For each cladding sub-layer, a torch traverses the tube to heat the tube to a temperature of 2050 °C. The SiCl₄ oxidizes into silica particles, which deposit on the interior wall of the tube by thermophoresis. The silica particles sinter to form a thin glass layer upon heating by the torch as the torch passes along the length of the tube. Multiple core sub-layers (in composite forming a core layer) are deposited next by flowing 1.2 to 3.6 g/min of SiCl₄ in 4000 to 5000 cc/min of O₂, 500 to 2000 cc/min of He, and 1000 to 5000 cc/min of N₂O. Also flowing in the tube is GeCl₄ in an amount that ranges from 0.04 to 0.72 g/min and POCl₃ in an amount that ranges from 0.03 to 0.09 g/min, depending on the desired index of the sub-layer being deposited. For each core sub-layer, the torch traverses the tube to heat the tube to a temperature of 1700 °C to 1900 °C. The SiCl₄, the GeCl₄, and the POCl₃ oxidize into silica (SiO₂), germania (GeO₂), and phosphorous oxide (P₂O₅) particles, respectively, which deposit on the cladding layer, on the interior surface of the glass tube, or on a previously formed core sub-layer by thermophoresis. The silica, germania, and phosphorous oxide particles sinter to form a thin layer of doped glass upon heating by the torch as the torch passes along the length of the tube. The tube with the cladding layer and the core layer deposited thereon is collapsed to produce a preform from which a glass fiber may be drawn. The amount of GeCl₄ flowing during each sub-layer deposition is controlled to produce a dopant concentration profile that gives rise to a near-parabolic refractive-index profile in a glass fiber drawn from the preform.

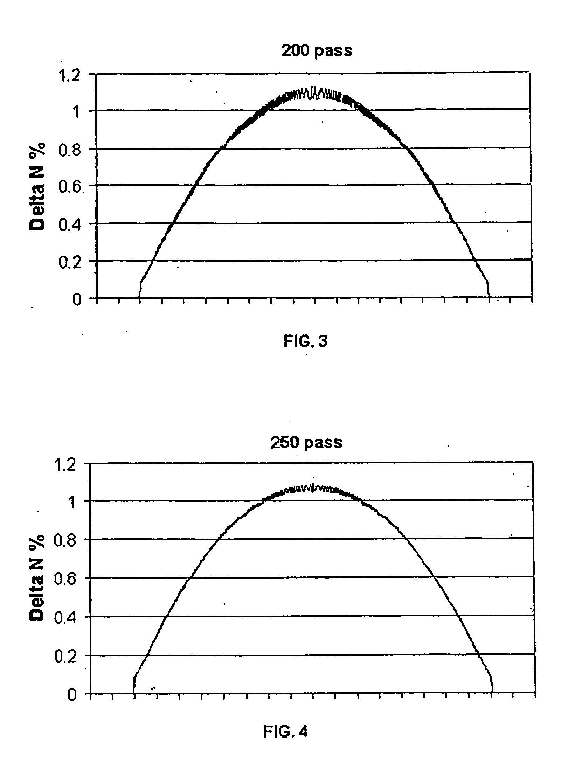

FIG. 3 is a chart showing the variation in index (refractive-index profile) along a radius of a core of a preform made by a conventional MCVD process. The core is formed of 200 sub-layers. As shown in the figure, the refractive-index profile generally has a near-parabolic shape defined with a distinct ripple structure.

FIG. 4 is a chart showing the variation in index along a radius of a core of another preform made by a conventional MCVD process. The core is formed of 250 sub-layers. As shown in the figure, the increase in the number of sub-layers causes a marked reduction in the amplitude of the ripple structure. However, the increase in the number of sub-layers also increases the time and the cost of making a preform.

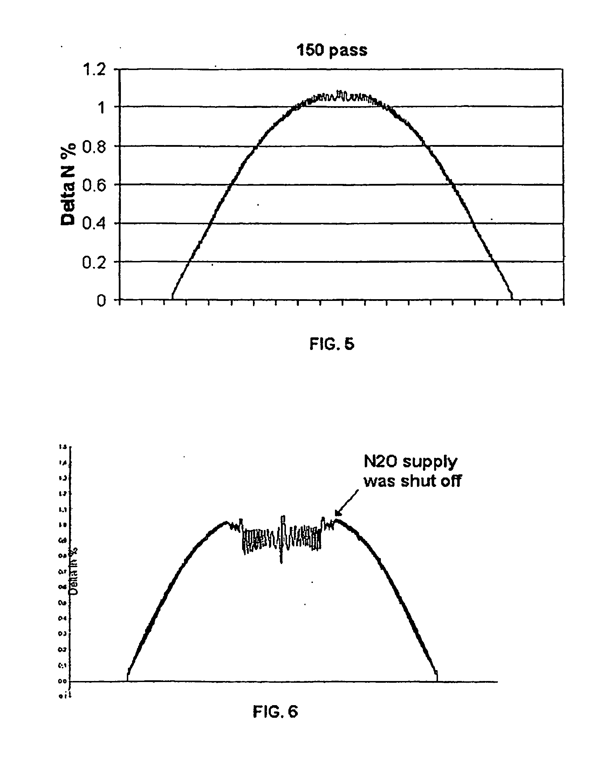

FIG. 5 is a chart showing the variation in index along a radius of a core layer of a preform made by an improved MCVD process according to the present invention. The core is formed of 150 sub-layers. As shown in the figure, the presence of N₂O during formation of the sub-layers results in a significant reduction in the amplitude of the ripple structure, such that the ripple structure is nearly as small as that shown in FIG. 4. That is, a layer formed by the improved MCVD process (i.e., using N₂O) and having only 150 sub-layers has a ripple structure as small as that of a core formed by a conventional MCVD process (i.e., without N₂O) and having 250 sub-layers.

FIG. 6 is a chart showing the variation in index along a radius of core of a preform made by an improved MCVD process according to the present invention, but in which the presence of N₂O during deposition of the sub-layers is turned off at the 124^{th} sub-layer. The total number of sub-layers is 150. As shown in the figure, the ripple structure is significantly greater at the center of the parabola, corresponding to the sub-layers deposited without the presence of N₂O, than at the outer regions of the parabola. This clearly demonstrates the effect of N₂O on producing a smoother refractive-index profile.

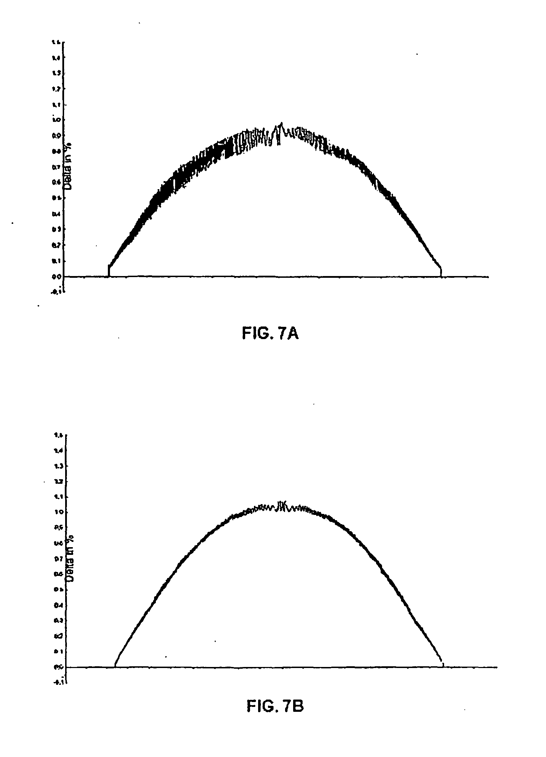

FIGS. 7A and 7B shows, respectively, a chart of the variation in index for a core formed of 150 sub-layers and made by a conventional MCVD process using a predefined deposition recipe, and a chart of the variation in index for a core layer formed of 150 sub-layers and made by an improved MCVD process, according to an embodiment of the present invention, using the same predefined deposition recipe. Note that in the case of the preform of FIG. 7B, the amount of O₂ flowing during deposition of the sub-layers was reduced to compensate for the oxygen atoms present from the addition of N₂O to the deposition process. As shown in the figures, the presence of N₂O during deposition of the core sub-layers significantly reduces the amplitude of the ripples in the refractive-index profile compared with the profile for the core made without N₂O.

Please note that, in FIGS. 3 through 7B, refractive-index defects were intentionally designed into the refractive-index profile of the core for experimental purposes.

As evident from the charts of FIGS. 3 through 7B, the improved MCVD process of the present invention may be used to produce high-yield, high-bandwidth GI MM fibers (suitable for 10 Gb/s transmission) because the improved MCVD process is able to produce preforms with a significantly smaller ripple structure than what is possible with the conventional MCVD process discussed above. In fact, while the rippling observed in cores produced by the conventional MCVD process may be reduced by increasing the number of sub-layers in the core (compare FIG. 3 with FIG. 4), the improved MCVD process enables a core with significantly reduced rippling to be produced with a reduced number of sub-layers (compare FIG. 3 and FIG. 5).
Thus, the improved MCVD process increases productivity by enabling fewer sub-layers to be used to achieve a core with a significantly smoother refractive-index profile.

The beneficial effects of the improved MCVD process of the present invention are believed to be the result of the addition of N₂O during deposition of the core sub-layers, which enhances oxidation of SiCl₄ and GeCl₄ without requiring a temperature increase, and which creates a "pre-reaction" region where soot particles form in a more uniform radial-temperature reaction zone. This leads to the production of smaller soot particles with a greater uniformity of GeO₂ within and between particles. It has been observed that with the improved MCVD process, i.e., when N₂O is present during deposition, soot formation occurs in a reaction zone that is upstream of the reaction zone observed for the conventional MCVD process. That is, with N₂O, soot formation is facilitated and occurs earlier in the process.

Further, because the soot particles begin to form sooner than in the conventional MCVD process, the particles spend more time in the hot zone. This allows for more frequent particle collisions and results in a better chemical or compositional homogenization. Also, because the particles have a longer residence time in the hot zone, they also reach a higher temperature. This should increase thermophoresis between the particles and the cooler temperature of the tube's wall, thus enhancing deposition and perhaps contributing to the reduced rippling in the parabolic refractive-index profile of the preforms.

Note that although U.S. Patent No. 6,109,065 to Atkins et al. discloses the use of N₂O and CIFO₃ (perchloryl fluoride) to produce more uniform soot layers, the uniformity addressed in that patent refers to structural uniformity, i.e., the uniformity of the porosity of the as-deposited soot layer (unsintered), which then undergoes solution doping followed by sintering. The Atkins et al. patent is silent regarding the effect of N₂O in reducing rippling in core sub-layers that are doped during deposition (i.e., does not undergo solution doping).

While the present invention has been described with respect to what is presently considered to be the preferred embodiment(s), it is to be understood that the invention is not limited to the disclosed embodiment(s). To the contrary, the invention is intended to cover various modifications and equivalent arrangements included within the spirit and scope of the appended claims. The scope of the following claims is to be accorded the broadest interpretation so as to encompass all such modifications and equivalent structures and functions.

As will be appreciated, there are many arrangements for practicing the improved MCVD process of the present invention. FIG. 2 illustrates only one of the many arrangements and in no way should it be construed that the present invention is limited to that arrangement.

## Claims

1. A method for reducing ripples in a refractive-index profile of a multimode optical fiber, the method comprising the steps of:
(a) providing a substrate;
(b) flowing along the substrate a gas mixture that includes O₂, a gaseous silicon-bearing compound, a gaseous dopant-bearing compound, and N₂O;
(c) moving a heat source along the substrate to cause the silicon-bearing compound and the dopant-bearing compound to form oxide soot particles, wherein the soot particles deposit as a first doped soot layer on the substrate;
(d) adjusting the gas mixture by adjusting a quantity of one or more of the O₂, the gaseous silicon-bearing compound, the gaseous dopant-bearing compound, and the N₂O flowing along the substrate; and
(e) moving the heat source along the substrate to cause the silicon-bearing compound and the dopant-bearing compound of the adjusted gas mixture to form oxide soot particles with a different dopant concentration, wherein the soot particles with the different dopant concentration deposit as a second doped soot layer on top of a first doped glass layer formed from the first doped soot layer,
wherein, when the heat source is moved along the substrate, a previously deposited soot layer is sintered to form a glass layer.

2. A method for reducing ripples in a refractive-index profile of a multimode optical fiber, the method comprising the steps of:
(a) providing a substrate;
(b) flowing along the substrate a gas mixture that includes O₂, a gaseous silicon-bearing compound, a gaseous dopant-bearing compound, and NF₃;
(c) moving a heat source along the substrate to cause the silicon-bearing compound and the dopant-bearing compound to form oxide soot particles, wherein the soot particles deposit as a first doped soot layer on the substrate;
(d) adjusting the gas mixture by adjusting a quantity of one or more of the O₂, the gaseous silicon-bearing compound, the gaseous dopant-bearing compound, and the NF₃ flowing along the substrate; and
(e) moving the heat source along the substrate to cause the silicon-bearing compound and the dopant-bearing compound of the adjusted gas mixture to form oxide soot particles with a different dopant concentration, wherein the soot particles with the different dopant concentration deposit as a second doped soot layer on top of a first doped glass layer formed from the first doped soot layer,
wherein, when the heat source is moved along the substrate, a previously deposited soot layer is sintered to form a glass layer.

3. A method for reducing ripples in a refractive-index profile of a multimode optical fiber, the method comprising the steps of:
(a) providing a substrate;
(b) flowing along the substrate a gas mixture that includes O₂, a gaseous silicon-bearing compound, a gaseous dopant-bearing compound, and CO;
(c) moving a heat source along the substrate to cause the silicon-bearing compound and the dopant-bearing compound to form oxide soot particles, wherein the soot particles deposit as a first doped soot layer on the substrate;
(d) adjusting the gas mixture by adjusting a quantity of one or more of the O₂, the gaseous silicon-bearing compound, the gaseous dopant-bearing compound, and the CO flowing along the substrate; and
(e) moving the heat source along the substrate to cause the silicon-bearing compound and the dopant-bearing compound of the adjusted gas mixture to form oxide soot particles with a different dopant concentration, wherein the soot particles with the different dopant concentration deposit as a second doped soot layer on top of a first doped glass layer formed from the first doped soot layer,
wherein, when the heat source is moved along the substrate, a previously deposited soot layer is sintered to form a glass layer.

4. A method according to any one of Claims 1, 2, and 3, further comprising the step of:
(f) repeating steps (d) and (e) to form a plurality of glass layers on the substrate; and
(g) producing a preform by collapsing the substrate with the plurality of glass layers formed thereon.

5. A method according to Claim 4, wherein the preform has a radial refractive-index profile with a near-parabolic shape.

6. A method according to Claim 4,
wherein a refractive index profile of the preform has a ripple structure with an amplitude smaller than a ripple structure of conventional preform made without N₂O, NF₃, or CO and made to have a same number of glass layers as the preform.

7. A method according to any one of Claims 1, 2, and 3, wherein the gaseous dopant-bearing compound is GeCl₄ and the gaseous silicon-bearing compound is SiCl₄.

8. A method according to any one of Claims 1, 2, and 3, wherein the first doped soot layer is deposited on a cladding layer on the substrate.

9. A method for reducing ripples in a refractive-index profile of a multimode optical fiber, the method comprising the steps of:
(a) providing a tubular substrate;
(b) flowing through the substrate a gas mixture that includes O₂, a gaseous silicon-bearing compound, a gaseous dopant-bearing compound, and N₂O;
(c) heating the gas mixture to cause the silicon-bearing compound and the dopant-bearing compound to form oxide soot particles, wherein the soot particles deposit as a doped soot layer on the substrate;
(d) sintering the doped soot layer to form a glass layer;
(e) adjusting the gas mixture by adjusting a quantity of one or more of the O₂, the gaseous silicon-bearing compound, the gaseous dopant-bearing compound, and the N₂O flowing along the substrate;
(f) heating the adjusted gas mixture to cause the silicon-bearing compound and the dopant-bearing compound of the adjusted gas mixture to form oxide soot particles with a different dopant concentration, wherein the soot particles with the different dopant concentration deposit as a doped soot layer on top of the glass layer; and
(g) repeating steps (d), (e), and (f) to form a plurality of glass layers on the substrate.

10. A method for reducing ripples in a refractive-index profile of a multimode optical fiber, the method comprising the steps of:
(a) providing a tubular substrate;
(b) flowing through the substrate a gas mixture that includes O₂, a gaseous silicon-bearing compound, a gaseous dopant-bearing compound, and NF₃;
(c) heating the gas mixture to cause the silicon-bearing compound and the dopant-bearing compound to form oxide soot particles, wherein the soot particles deposit as a doped soot layer on the substrate;
(d) sintering the doped soot layer to form a glass layer;
(e) adjusting the gas mixture by adjusting a quantity of one or more of the O₂, the gaseous silicon-bearing compound, the gaseous dopant-bearing compound, and the NF₃ flowing along the substrate;
(f) heating the adjusted gas mixture to cause the silicon-bearing compound and the dopant-bearing compound of the adjusted gas mixture to form oxide soot particles with a different dopant concentration, wherein the soot particles with the different dopant concentration deposit as a doped soot layer on top of the glass layer; and
(g) repeating steps (d), (e), and (f) to form a plurality of glass layers on the substrate.

11. A method for reducing ripples in a refractive-index profile of a multimode optical fiber, the method comprising the steps of:
(a) providing a tubular substrate;
(b) flowing through the substrate a gas mixture that includes O₂, a gaseous silicon-bearing compound, a gaseous dopant-bearing compound, and CO;
(c) heating the gas mixture to cause the silicon-bearing compound and the dopant-bearing compound to form oxide soot particles, wherein the soot particles deposit as a doped soot layer on the substrate;
(d) sintering the doped soot layer to form a glass layer;
(e) adjusting the gas mixture by adjusting a quantity of one or more of the O₂, the gaseous silicon-bearing compound, the gaseous dopant-bearing compound, and the CO flowing along the substrate;
(f) heating the adjusted gas mixture to cause the silicon-bearing compound and the dopant-bearing compound of the adjusted gas mixture to form oxide soot particles with a different dopant concentration, wherein the soot particles with the different dopant concentration deposit as a doped soot layer on top of the glass layer; and
(g) repeating steps (d), (e), and (f) to form a plurality of glass layers on the substrate.

12. A method according to any one of Claims 9, 10, and 11, further comprising the step of:
(h) producing a preform by collapsing the substrate with the plurality of glass layers formed thereon.

13. A method according to Claim 12, wherein the preform has a radial refractive-index profile with a near-parabolic shape.

14. A method according to Claim 12,
wherein a refractive index profile of the preform has a ripple structure with an amplitude smaller than a ripple structure of conventional preform made without N₂O, NF₃, or CO and made to have a same number of glass layers as the preform.

15. A method according to any one of Claims 9,10, and 11, wherein the gaseous dopant-bearing compound is GeCl₄ and the gaseous silicon-bearing compound is SiCl₄.

16. A multimode optical fiber formed using the method of any one of Claims 1, 2, and 3.

17. A multimode optical fiber formed using the method of any one of Claims Claim 9, 10, and 11.
